Europäisches Patentamt

European Patent Office

Office européen des brevets

(11) Publication number: **0 242 914 B1**

## EUROPEAN PATENT SPECIFICATION

(45) Date of publication of patent specification: **15.07.92**

(51) Int. Cl.5: **G06F 15/336**, G01S 7/52, //A61B8:06

(21) Application number: **87200687.9**

(22) Date of filing: **13.04.87**

(54) Correlator circuit and device for ultrasound flow mapping comprising such a circuit.

(30) Priority: **21.04.86 US 854260**
**21.04.86 US 854379**

(43) Date of publication of application:
**28.10.87 Bulletin 87/44**

(45) Publication of the grant of the patent:
**15.07.92 Bulletin 92/29**

(84) Designated Contracting States:
**DE ES FR GB SE**

(56) References cited:

**IEEE TRANSACTIONS ON ACOUSTICS, SPEECH, AND SIGNAL PROCESSING, vol. ASSP-24, no.4, August 1976, pages 320-327; C.H. KNAPP et al.: "The generalized correlation method for estimation of time delay"**

**IEEE TRANSACTIONS ON ACOUSTICS, SPEECH, AND SIGNAL PROCESSING, vol. ASSP-32, no. 2, April 1984, pages 371-380, IEEE; S.-H. LEUNG et al.: "State-space realizations of fractional-step delay digital filters with applications to Array beamforming"**

(73) Proprietor: **North American Philips Corporation**
**100 East 42nd Street 9th Floor**
**New York, N.Y. 10017(US)**

(72) Inventor: **Barnes, Casper William**
**c/o INT. OCTROOIBUREAU B.V. Prof. Holstlaan 6**
**NL-5656 AA Eindhoven(NL)**

(74) Representative: **Veenstra, Gustaaf et al**
**INTERNATIONAAL OCTROOIBUREAU B.V.**
**Prof. Holstlaan 6**
**NL-5656 AA Eindhoven(NL)**

**Description**

The invention relates to a correlator circuit for determining the cross-correlation of a first input signal and a second input signal, said input signals each being represented as a series of discrete digital samples having a sample period $\tau$.

The invention further relates to a device for determining parameters of movement in a media by ultrasound pulse-echo measurements, comprising at least one ultrasound transducer, means for exciting emission of a pulsed echo sound signal from said transducer at a pulse repetition frequency $F = 1/T$, means for receiving ultrasound echoes which are reflected from said media back to said transducer and for processing such signals in a digital processing channel which acts on discrete samples of A-line signals received from said transducer and comprises, in cascade, means for suppressing signals in said A-lines which originate from fixed regions in said media, flow parameter estimation means, discriminator means, and means for displaying estimates of flow parameters.

In pulse-echo ultrasound investigations, the velocity of ultrasound scatterers can be estimated from the relative time delay between signals in successive A-lines. This relative time delay can be determined from the value of a time lag which maximizes the cross-correlation of signals from two successive A-lines.

The use of correlation functions makes it possible to measure time delays between signals as described, for example, in the article "The Generalized Correlation Method for Estimation of Time Delays", C.H. Knapp and G.C. Carter, IEEE Transactions on Acoustics, Speech, and Signal Processing, Vol. ASSP-24, No. 4, 1976.

If the ultrasound scatterers have low velocity, the relative time delay between the signals in successive A-lines may be much shorter than the pulse repetition rate of the ultrasound transmitter (that is: the time interval between signal samples). Conventional discrete time correlators only generate correlation estimates for time lags which are multiples of the signal sample interval. Thus, when the scatterer velocity is low, it is difficult to accurately locate the maximum of the cross-correlation function from cross-correlation samples that are spaced at multiples of the sample interval. This problem is particularly severe if the cross-correlation estimates are computed with one-bit correlators.

It is an object of the invention to provide a correlator circuit that overcomes the above-mentioned difficulty.

The correlator circuit according to the invention is characterized in that it comprises a plurality of fractional step delay (FSD) digital interpolation filters, each of said filters functioning to interpolate the value of a filter input signal at a different predetermined time between discrete signal sample times, and a plurality of discrete time correlation estimator circuits connected in cascade with said interpolation filters.

The implementation and use of fractional-step delay (FSD) digital filters is described in the article "State Space Realization of Fractional-Step Delay Digital Filters with Applications to Array Beam Forming", Shu-Hung Leung and Casper W. Barnes, IEEE Transactions on Acoustics, Speech, and Signal Processing, Vol. ASSP-32, No. 2, April 1984, pages 371-380.

According to the invention a device for determining the parameters of movement in media of the kind set forth in the preamble is characterized in that the flow estimation means comprise a correlator circuit according to the invention.

Fractional step delayed signal estimates are correlated, preferably using one-bit correlators, to locate the maximum of the cross-correlation function of the A-line signals. Fractional step correlators are relatively simple devices which are implementated with the full desired signal precision, that is 8, 12 of 16 data bits. Once fractional step delay estimates have been generated using the FSD filters, the cross-correlations of the full precision data can be efficiently computed with the data limited to one-bit.

When using FSD digital filters, the complexity of hardware used to implement an ultrasound velocity measuring scanner can be reduced if the A-line signal is interpolated prior to correlation. The multiplications required for the FSD filters can be efficiently implemented by a table look-up using ROMs.

A description of the prior art and a detailed description of an embodiment of the present invention is made with reference to the appended drawings in which:

Figure 1 is an ultrasound, time domain flow imaging scanner of the prior art;

Figure 2 is a fixed echo suppressor of the prior art;

Figure 3 is a 1-bit correlator of the prior art; and

Figure 4 is a discriminator of the prior art.

Figure 5 represents a linear interpolation function;

Figure 6 represents the application of the function of Figure 1 to interpolate a signal;

Figure 7 represents a sinc interpolation function;

Figure 8 represents the application of the function of Figure 3 to interpolate a signal;

Figure 9 represents the truncated, delayed unit pulse response of a sinc (k ($\tau$)) filter,
Figure 10a and 10b are alternate block diagram representations of the interpolator;
Figure 11 is a fractional step-delay digital filter using a transversal filter architecture;
Figure 12 is a block diagram of a correlation estimator;
Figure 13 is a fractional step correlation estimator;
Figure 14a is a fractional step cross-correlator;
Figure 14b illustrates the application of the correlator of Figure 10A to ultrasound flow imaging.

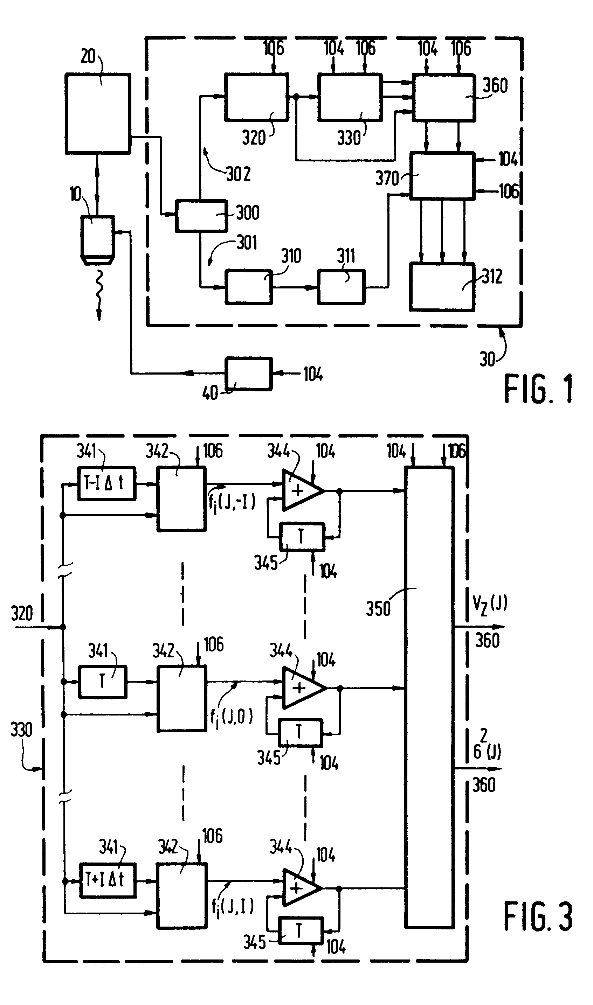

European patent application (Publication Number EP-A-0225667) having priorities of 3.12.85 and 25.3.86 is a document in accordance with Article 54(3)EPC and describes an instrument as shown in Figure 1 for producing images of fluid flow within a body by ultrasound pulse-echo techniques (especially of blood-flow in organs such as the heart). It comprises at least one ultrasound transducer 10 associated with a pulse generator which generates a periodic pulses signal at a pulse repetition frequency $F = \frac{1}{T}$ . The transducer 10 is connected to a transmitter stage 20, a reception and signal processing stage 30, and a device 40 that effects mechanical scanning of the transducer. The transmitter stage 20 comprises a generator of electrical excitation signals that are sent toward the transducer 10 which converts them into periodic pulse trains of ultrasound energy. This emission is commanded by clock signals at the predetermined frequency F - on the order of, for example 5 kHz - by a sequencer comprising, an oscillator having a frequency of, for example, 32 megahertz, and a frequency divider. The divider delivers clock signals as well as other command signals to connections 104 and 106 respectively, at 1 kilohertz and 16 megahertz in the example described here. A T-R switch prevents the blinding of the reception circuits by the emission signals.

The reception and processing stage 30 comprises, at the output of the T-R switch, a high-frequency amplifier 300 (which includes means for compensation of gain as a function of the depth) followed by two parallel signal processing channels 301 and 302. Channel 301 is the conventional type, and comprises, in series, an envelope detector 310, a logarithmic compression amplifier 311, a storage and scanning conversion device 370 (which also includes a color coding function), and a display device 312. This channel 301 forms images of the media explored, in a gray scale, based on the principles of classic echography.

Channel 302 comprises, in series, a fixed echo suppression circuit 320, a flow parameter estimation circuit 330, a discriminator circuit 360, the storage, scanning conversion and color coding device 370, and the display device 312.

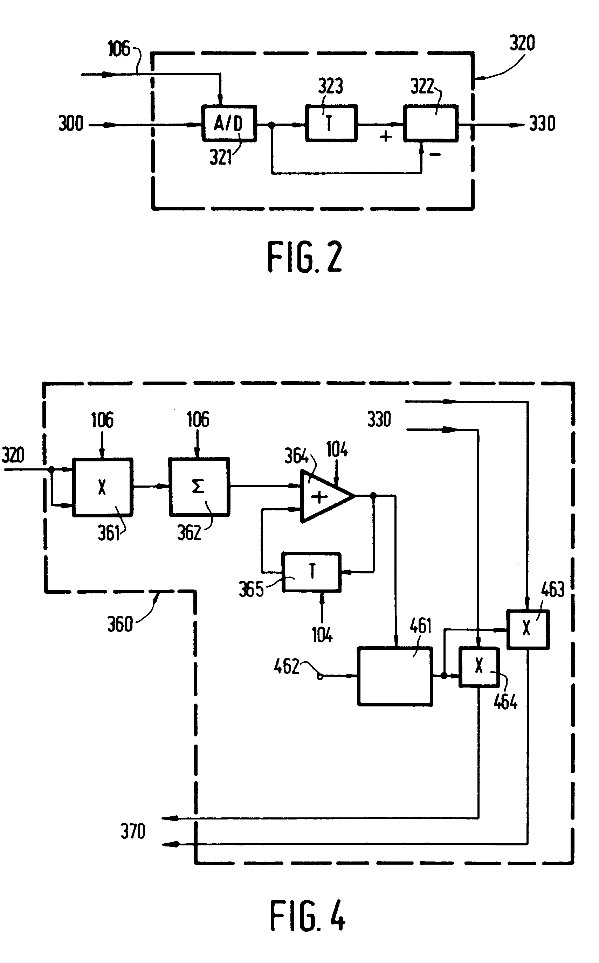

In Figure 2 the digital fixed-echo suppression circuit 320 comprises an analog-digital converter 321 whose output is connected directly to the negative input of a subtractor 322 and through a delay circuit 323 to the positive input of the same subtractor. The delay of circuit 323 can be equal to T. Circuit 320 eliminates all fixed echoes, especially those caused by the reflection of the ultrasound energy from the walls of the vessels which contain the flows under study. Fixed echoes are annoying due to their much higher amplitude (on the order of +40 dB in the case of blood flows) than the working signals which are back-scattered by moving targets. Circuit 320 is commanded, through connection 106, by the frequency divider of the sequencer that provides it with the sampling command signal at the 16 MHz frequency.

Figure 3 shows the flow parameter estimation circuit 330. It comprises correlation circuits and an interpolation circuit. On the bases of the difference between two successive echographic A-lines of samples $d_i(t)$, $d_{i+1}(t)$, etc ... (where i represents the index of this signal) successively furnished by the fixed echo suppression circuit 320, the correlation circuit delivers an odd number (2I + 1) of correlation function values. The interpolation circuit thus delivers parameters which characterize the various flows encountered along the axis of propagation of the ultrasonic wave on the basis of these values. These parameters are here the axial components of the average local speed $V_z$ and the local variance $\sigma2$ of the latter, ("local" here being used in the sense of the localization in depth along the axis of propagation.)

The correlation circuits comprise (2I + 1) correlators 342 which directly receive the output $d_{i+1}(t)$ of feed echo suppression circuit 320 and, at a second input, receive this same output delayed by delays 341 and therefore corresponding to the preceding signal $d_i(t)$. Furthermore, each of delays 341 has a distinct delay which assumes (2I + 1) values from T - I$\Delta$t to T + I$\Delta$t, where $\Delta$t is the sampling interval to permit computing the (2I + 1) values of the correlation function. This parallel computation of the (2I + 1) values of the correlation function uses K successive samples of the two input signals of the correlators. The groups of K samples define successive time windows of length K$\Delta$t offset gradually at the cadence of the frequency imposed by connection 106. The correlation function is defined by an expression of the type:

$$f_i (J,P) = \sum_{k=1}^{k=K} d_i((k + J)\Delta t) \cdot d_{i+1}((k+J+P)\Delta t) \quad (1)$$

in which:

- J determines the start of the time window of length $K\Delta t$,
- P is the time delay introduced bwtween $d_i$ and $d_{i+1}$ ranging from -I to +I,
    i is the rank of the difference between two successive echographic lines $e_i$ and $e_{i+1}$.

The correlators 342, commanded through output connection 106 of the frequency divider of the sequencer, are preferably 1-bit correlators (for example type TDC 1023 made by TRW, La Jolla CA 92038). When the embodiment described comprises these 1-bit correlators, the interpolation circuit 350 is then, in general, a linear interpolation circuit.

The interpolation circuit 350 can be a programmed microprocessor, or preferably a wired computer unit. This interpolation circuit works as follows: in a first stage there is a search for the maximum value among the (2I + 1) values of the correlation function, the two adjacent correlation function values are associated with it, and these three values permit the reconstitution of the principal correlation peak, in isoceles form. The abscissa $\tau(J)$ of the principal peak of correlation gives access to the local speed $V_z$ at depth

$$z_O = \frac{cJ\Delta t}{2} \tag{2}$$

by multiplication according to the formula:

$$V_z(z_O) = c \cdot \frac{\tau(J)}{2T} \tag{3}$$

and the amplitude $f_{MAX}$ of this peak gives access to the variance

$$\sigma^2(z_O)$$

by operating according to the formula:

$$\sigma^2(z_O) = A(1 - \frac{f_{MAX}(J)}{K}) \tag{4}$$

where A is a factor of proportionality.

Between each of the correlators 342 and the corresponding inputs of the interpolation circuit 350 there is an average computing circuit (which is in fact an accumulator), each comprising an adder 344 and a delay line 345 with a delay T. These average computing circuits permit the accumulation of the correlation function values on N successive A-lines, and to find their average. The adders 344 and the delay lines 345 are connected to the sequencer by connection 104 to be reset to zero at regular (N x T) intervals.

The output signals from the flow parameter estimation circuit 330 are then validated by a discriminator circuit 360. The values thus confirmed are sent toward the display device 312 by means of color coding device 370.

The presence of the discriminator circuit 360 (shown in Fig. 4) is essential. Outside of flow zones the output signal from fixed echo suppression circuit 320 is noise and the output of the flow parameter estimation circuit 330, which processes this noise, is not an indication of a zero speed. Circuit 360 therefore comprises, in series, a multiplier 361 which receives and squares the output signal $d_i$ from the feed echo suppression circuit 320; a summor 362 which computes the local energy of this difference signal according to the formula:

4

$$E_i(J) = \sum_{k=1}^{k=K} d_i 2((k+J)\Delta t); \qquad (5)$$

a circuit (364, 365) which computes the average of the local energy on N shots, (as in the case of circuit 344 and 345 it is an accumulator comprising an adder 364 and a delay line 365 of delay T), that is to say (N-1) differences according to the expression:

$$E(J) = \sum_{i=1}^{i=N-1} E_i(J). \qquad (6)$$

The average value computer is followed a validation circuit which comprises a comparator 461 which receives on a first input the output from the accumulator 364, 365 and on a second input 462a reference voltage forming a threshold. The output from the comparator 461 is the logic level 0 or 1 depending on whether the voltage from the accumulator is below or above the reference threshold. Two multipliers 463 and 464 receive, respectively, the output signals from circuit 330 at their first input, and transmit these two signals, hereinafter V'$_z$ and $\sigma$'2 at their respective outputs or simply transmit zero values depending on whether the validation signal delivered at their second input by comparator 461 is respectively 1 or 0. Outside the true flow zones, the average energy computed at the output from circuit 364, 365 is that of noise alone, and can be measured in the absence of excitation, to determine the appropriate value of the threshold. In the presence of signals back-scattered by the moving targets, the average energy of the signal d$_i$ is higher than that of the noise alone; this validates signals delivered by flow parameter estimation circuit 330.

The two outputs of the discriminator circuit 360 are sent towards the storage, scanning conversion and color coding device 370.

In accordance with the invention the flow estimation circuit comprises a correlator circuit comprising FSD interpolation filters and discrete time correlators which will now be explained in detail.

FSD Digital Interpolation Filter

Let x (n$\tau$) denote digital samples of a signal taken with a sample period $\tau$. The signal values between sample times can be approximated by using an appropriate interpolating function in the following form:

$$X(t) = \sum_n X(n\tau) \; g(t-n\tau)$$

$$= \ldots + X(-2\tau) \; g(t+2\tau) + x(-\tau) \; g(t+\tau) \qquad (7)$$
$$+ x(0) \; g(t) + x(\tau)g(t-\tau) + \ldots$$

where g(t) is an interpolating function.

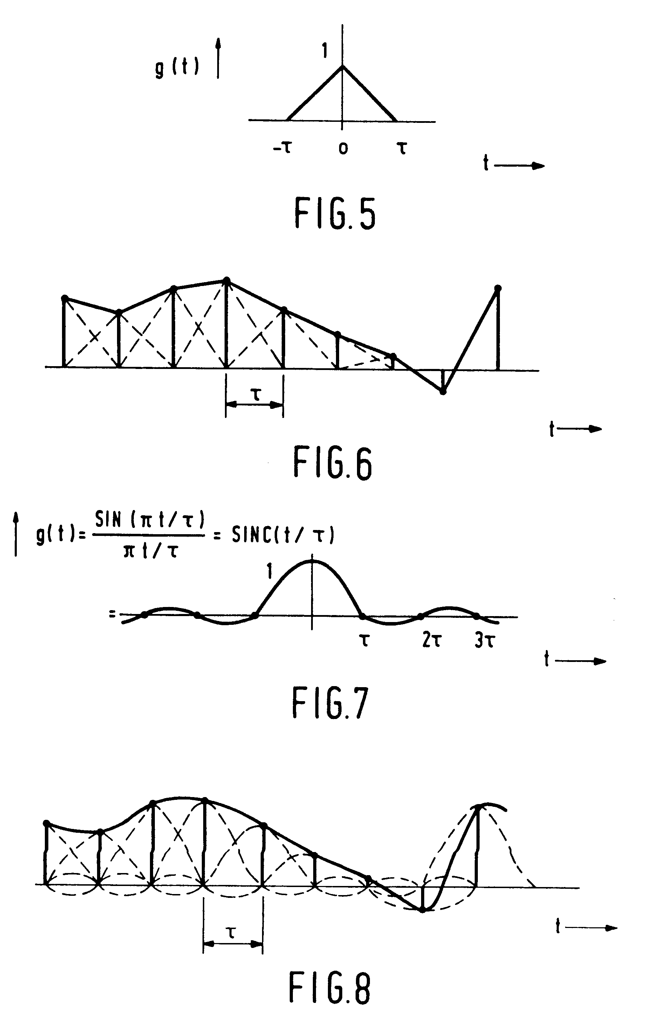

Figure 5 illustrates a linear interpolating function g(t) which has values of 0 for a t = ± $\tau$ and a value of 1 for t = 0. In this case the interpolated function comprises a series of straight line approximations as illustrated in Figure 6.

The linear interpolating function of Figure 5 requires infinite bandwidth. For band limited interpolation let

$$g(t) = \frac{SIN(\pi t/\tau)}{t/\tau} = sinc(t/\tau) \qquad (8)$$

In this case the interpolating function g(t) has the form illustrated in Figure 7 and the interpolated signal will have the form illustrated in Figure 8.

A time delay interpolator can be constructed by taking samples of x(t) with sample period $\tau$ and with sample times that are offset by a fraction of $\xi$ from the original samples; that is

$$y(m\tau) = x(m\tau-\xi) \qquad (9)$$

where

$$0 < \xi < \tau. \qquad (10)$$

These samples can be obtained from the interpolated signal using equation 11:

$$y(m\tau) = x(m\tau - \xi) = \sum_n x(n\tau) \, g(m\tau - n\tau - \xi) \qquad (11)$$

Let

$$h_\xi(n) = g(n\tau - \xi) \qquad (12)$$

then

$$y(m\tau) = \sum_n x(n\tau) \, h_\xi(m-n) = \sum_n h_\xi(n) \, x(m\tau - n\tau) \qquad (13)$$

Thus, the interpolated samples can be obtained from the original samples by a discrete convolution which represents a time-invariant filtering operation

$$y(n\tau) = x(n\tau) \cdot h(n) \qquad (14)$$

where h(n) is the unit pulse response of the interpolating filter.

To ensure that h(n) is finite and causal, it may be necessary to truncate the interpolating function and to introduce a delay $K\tau$; thus, in practice the interpolating filter should compute

$$y(m\tau) = x(m\tau - K\tau - \xi) \qquad (15)$$

where K is a positive integer chosen to be sufficiently large so that

$$g(n\tau - K\tau - \xi) = 0 \qquad \text{for } n<0. \qquad (16)$$

Thus the filtering operation for the time delay interpolator is

$$y(m\tau) = x(m\tau - K\tau - \xi)$$

$$= \sum_{n=0}^{N-1} h_\xi(n) \, x(n\tau - m\tau) \qquad (17)$$

where

$$h_\xi(n) = g(n\tau - K\tau - \xi). \qquad (18)$$

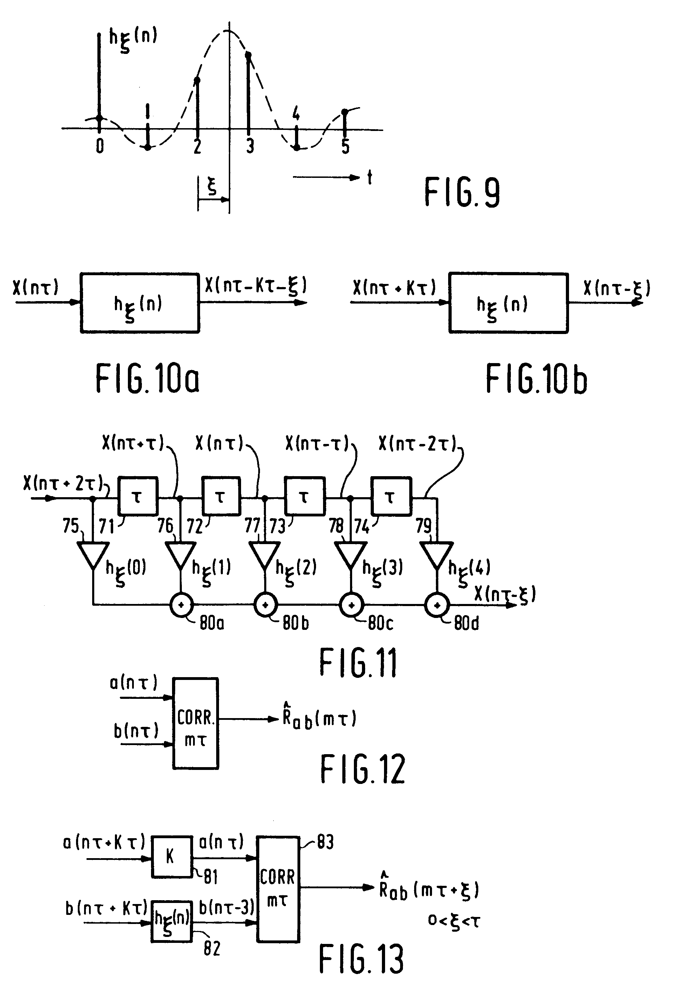

Figure 9 illustrates the truncated pulse response of a sinc $(K\tau)$ interpolator for the case n = 6 and K = 2. Figure 10a and 10b are alternate representations of the transfer function of such an interpolator.

Figure 11 illustrates the implementation of the FSD filter using a conventional transversal filter architecture with n = 5. The input signal passes through cascade delay elements 71, 72, 73, and 74 each having a delay period $\tau$. The delay cascade is tapped and signals from the successive delays are multiplied by factors $h_\xi(0)$, $h_\xi(1)$, $h_\xi(2)$, $h_\xi(3)$, and $h_\xi(4)$ in multipliers 75, 76, 77, 78, and 79 respectively. The output signals from the multiplier 75 through 79 are summed in adding circuits 80a, 80b, 80c, and 80d to produce the filter output. The multiplying factors are calculated using equations 17 and 18 and standard digital filter design techniques as described in the above-mentioned background references. The FSDfilter is typically constructed to match the precision of the input signal, for example 8, 12, or 16-bit data. For 8 or 12-bit data the multipliers can be efficiently implemented by table look-up with ROMS.

Discrete-Tome Correlators

The cross-correlation between two discrete-time signals, $a(n\tau)$ and $b(n\tau)$, can be estimated from time averages of the form

$$\hat{R}_{ab}(m\tau) = C \sum_n a(n\cdot\tau + m\tau)\ b(n\tau) \qquad (19)$$

where the sum is taken over a finite number of terms and C is an appropriate normalizing constant. A discrete correlation estimator can be represented in block diagram form as shown in Figure 12. Figure 13 illustrates a fractional step correlation estimator. A first signal $a(n\tau + K\tau)$ is passed through a constant delay circuit 81 with delay time K. The second input signal $b(n\tau + K\tau)$ is passed through FSD interpolation filter 82 with transfer function $h_\xi(n)$. The outputs of circuits 81 and 82 are applied to inputs of a discrete time correlation estimator 83 the magnitude of whose output is an estimate of the cross-correlation between the signals $a(n\tau)$ and $b(n\tau - \xi)$.

Figure 14a is an example of a fractional step cross-correlator 98. A first signal $a(n\tau + K\tau)$ is applied to a first input of a fixed delay circuit 84 having a delay time $K\tau$. The second input signal $b(n\tau + K\tau)$ is applied to the input of a second delay circuit 85 which is identical to the delay circuit 84. The second input signal is also applied to the inputs of a number of (in this example three) FSD interpolation filters 86, 87, and 88 having, for example, respective delay times of $\tau/4$, $\tau/2$, and $3\tau/4$. The output of delay circuit 84 is separately correlated with the output of delay circuit 85 and with the outputs of the fractional step interpolation filters 86, 87, and 88 in discrete time correlation estimator circuits 89, 90, 91 and 92 whose estimates respectively represent the cross-correlation of the input signals at times 0, $\tau/4$, $\tau/2$, $3\tau/4$. The same inputs are respectively applied to discrete time correlation estimators 93, 94, 95 and 96 which are constructed to cross-correlate signals spaced by sample interval - $\tau$ and whose outputs respectively represent estimates of the cross-correlation of the signals at period $-\tau$, $-3\tau/4$, $-\tau/2$, and $-\tau/4$. The outputs of delay circuits 84 and 85 are further applied to a single discrete time correlation estimator 97 having an integral delay of $\tau$ whose output is an estimate of the correlation of the input signals at time $\tau$. The respective outputs of the correlation estimators 89-96 represent the cross-correlation function of the input signals.

Although fractional step delays of $\tau/4$, $\tau/2$ and $3\tau/4$ were used in this embodiment, FSD filters can be designed to produce fractional step delays with any value between zero and $\tau$(where $\tau$ is the sample period). For example, one could use N FSD filters with any N different values of delay.

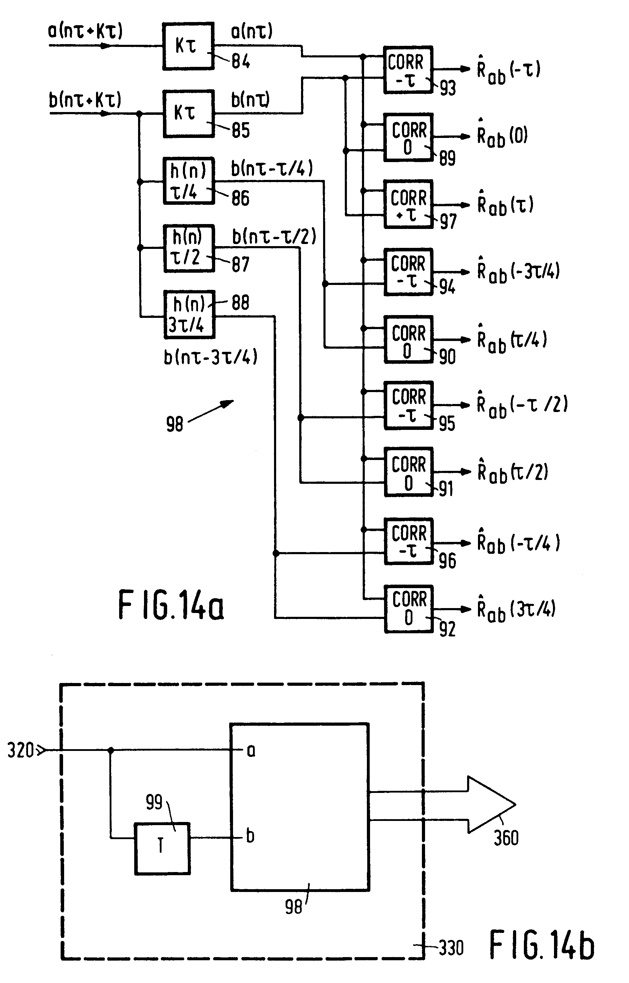

Figure 14b illustrates the application of the fractional step correlator in a flow estimator 330 for use in the prior art ultrasonic pulse echo flow measuring device of Figure 1. The output of the fixed echo suppressor 320 is applied to the first input of a fractional step correlator 98 and to the input of a fixed delay 99 having a delay period T equal to the pulse repetition period of the ultrasound transmitter 20. The output of the delay 99 is applied to the second input of the fractional step correlator 98 so that the respective inputs of the fractional step correlator represents successive ultrasound echo A-lines with fixed echoes suppressed. The output signals from the correlation estimators in the fractional step correlator 98 are applied, through the discriminator 360 and the scan converter and color coder 370 to determine the color of regions in the display 312, as in the prior art scanner.

Although the present invention has been described as a cascade fractional step delay interpolation filters followed by 1-bit correlation estimators, it is also possible to construct the fractional step correlator in a manner similar to that indicated in the prior art Figure 3 with fractional step interpolation filters acting on the output of correlation estimators.

**Claims**

**1.** A correlator (98) circuit for determining the cross-correlation of a first input signal (a) and a second input signal (b), said input signals each being represented as a series of discrete digital samples ($n\tau$ + $k\tau$) having a sample period $\tau$, characterized in that it comprises

a plurality of fractional step delay FSD digital interpolation filters (86,87,88), each of said filters functioning to interpolate the value of input signal at a different predetermined time between said second discrete samples, and

a plurality of discrete time correlation estimator circuits (89-93) connected in cascade with said interpolation fiters so that each of said circuits (85-93) determines the correlation between said first signal and an output of one of said interpolation filters.

**2.** A correlator circuit as claimed in claim 1 characterized in that said first input signal is applied to a first input of each of said correlation estimators, said second input signal being applied to the input of each of said FSD interpolation filters, and the output of each said FSD filters being applied to second inputs of corresponding ones of said discrete time correlation estimators.

**3.** A correlator circuit as claimed in claim 1, characterized in that it further comprises

first fixed delay means, having a delay period $K\tau$, said first input signal being applied to the input of said first delay means and second fixed delay means having a delay period $K\tau$, said second input signal being connected to the input of said second delay means, each of said FSD interpolation filters being connected to receive samples of said second input signal and to generate an estimate of the value of said second signal at a different time between the discrete samples times of said signal, so that each of said FSD filters estimates the value of said second signal at a separate time between said discrete sample times and each of said discrete time correlation estimator circuits having a first input connected to receive delayed samples of the first signal from the output of said first fixed delay means and having a second input connected to receive one of the estimates of the value of said second signal from a corresponding output of either said second delay means or one of said interpolation filters, to generate therefrom an estimate of the cross-correlation between the inputs supplied to said first and second inputs.

**4.** A correlator circuit as claimed in anyone of claims 1, 2 or 3, characterized in that said discrete time correlation estimator circuits are one-bit correlators.

**5.** A correlator circuit as claimed in anyone of the preceding claims, characterized in that the said fractional step delay interpolation filters are constructed to interpolate the value of said second input signal at a set of intervals which are fractions of the sample period $\tau$ after said discrete sample times of said second signal.

**6.** A correlator circuit as claimed in claim 5 characterized in that it comprises N-1 fractional step delay interpolation filters which are constructed to interpolate the value of said second input signal at intervals of $\frac{K\tau}{N}$ after said discrete sample times of said second signal where N is an integer greater than 1 and k = 1, 2, 3, ... N-1.

**7.** A device for determining parameters of movement in a media by ultrasound pulse-echo measurements, comprising at least one ultrasound transducer, means for exciting emission of a pulsed echo sound signal from said transducer at a pulse repetition frequency F = 1/T, means for receiving ultrasound echoes which are reflected from said media back to said transducer and for processing such signals in a digital processing channel which acts on discrete samples of A-line signals received from said transducer and comprises, in cascade, means for suppressing signals in said A-lines which originate from fixed regions in said media, flow parameter estimation means, discriminator means, and means for displaying estimates of flow parameters, characterized in that said flow estimation means comprise a correlator circuit as claimed in anyone of the preceding claims.

**Revendications**

**1.** Circuit corrélateur (98) destiné à déterminer la corrélation croisée entre un premier signal d'entrée (a) et un second signal d'entrée (b), ces signal d'entrée étant chacun représentés par une série d'échantillons numériques discrets ($n\tau$ + $K\tau$) présentant une période d'échantillonnage $\tau$, caractérisé

en ce qu'il comprend :

une pluralité de filtres d'interpolation numériques à retard par pas fractionnels FSD (86, 87, 88), chacun de ces filtres intervenant pour interpoler la valeur dudit second signal d'entrée à un instant prédéterminé différent entre lesdits échantillons discrets, et

une pluralité de circuits estimateurs de corrélation de temps discrets (89 à 93) connectés en cascade avec ces filtres d'interpolation de sorte que chacun des circuits (85 à 93) détermine la corrélation entre le premier signal et une sortie de l'un desdits filtres d'interpolation.

2. Circuit corrélateur suivant la revendication 1, caractérisé en ce que le premier signal d'entrée est appliqué à une première entrée de chacun des estimateurs de corrélation, le second signal d'entrée étant appliqué à l'entrée de chacun des filtres d'interpolation FSD et la sortie de chacun des filtres FSD étant appliquée aux secondes entrées d'estimateurs de corrélation de temps discrets correspondants.

3. Circuit corrélateur suivant la revendication 1, caractérisé en ce qu'il comprend, en outre :

des premiers moyens à retard fixe accusant une période de retard $K\tau$, le premier signal d'entrée étant appliqué à l'entrée desdits premiers moyens à retard et des seconds moyens à retard fixe accusant une période de retard $K\tau$, le second signal d'entrée étant appliqué à l'entrée des seconds moyens à retard, chacun des filtres d'interpolation FSD étant connecté de manière à recevoir des échantillons du second signal d'entrée et à produire une estimation de la valeur de ce second signal à un instant différent entre les instants d'échantillonnage discrets de ce signal de sorte que chacun des filtres FSD estime la valeur du second signal à un instant séparé entre les instants d'échantillonnage discrets et chacun des circuits estimateurs de corrélation de temps discrets présentant une première entrée connectée de manière à recevoir des échantillons retardés du premier signal de la sortie des premiers moyens à retard fixe et présentant une seconde entrée de manière à recevoir l'une des estimations de la valeur du second signal d'une sortie correspondante des seconds moyens à retard ou de l'un des filtres d'interpolation pour produire, à partir de celle-ci, une estimation de la corrélation croisée entre les entrées fournies à la première et à la seconde entrée.

4. Circuit corrélateur suivant l'une quelconque des revendications 1, 2 ou 3, caractérisé en ce que les circuits estimateurs de corrélation de temps discrets sont des corrélateurs 1 bit.

5. Circuit corrélateur suivant l'une quelconque des revendications précédentes, caractérisé en ce que les filtres d'interpolation à retard par pas fractionnels sont construits de manière à interpoler la valeur du second signal d'entrée pour une série d'intervalles qui sont des actions de la période d'échantillonnage $\tau$ après les instants d'échantillonnage discrets du second signal.

6. Circuit corrélateur suivant la revendication 5, caractérisé en ce qu'il comprend N-1 filtres d'interpolation à retard par pas fractionnels qui sont construits de manière à interpoler la valeur du second signal d'entrée à des intervalles de $K\tau/N$ après les instants d'échantillonnage discrets du second signal, où N est un nombre entier supérieur à 1 et K = 1,2,3,...,N-1.

7. Dispositif pour déterminer des paramètres de mouvement dans un milieu par des mesures d'échos d'impulsions ultrasonores comprenant au moins un transducteur à ultrasons, des moyens pour exciter l'émission d'un signal d'écho sonore pulsé provenant du transducteur à une fréquence de répétition d'impulsions F = 1/T, des moyens pour recevoir des échos ultrasonores qui sont réfléchis par le milieu vers le transducteur et pour traiter ces signaux dans un canal de traitement numérique qui agit sur des échantillons discrets de signaux de ligne A reçus du transducteur et qui comprend, en cascade, des moyens pour supprimer, dans les lignes A, des signaux qui proviennent de régions fixes du milieu, des moyens d'estimation de paramètres de flux, des moyens de discrimination et des moyens pour afficher des estimations de paramètres de flux, caractérisé en ce que les moyens d'estimation de flux comprennent un circuit corrélateur suivant l'une quelconque des revendications précédentes.

**Patentansprüche**

1. Korrelator-(98)-Schaltung zum Bestimmen der Kreuzkorrelation eines ersten Eingangssignals (a) und eines zweiten Eingangssignals (b), wobei die Eingangssignale je als eine Reihe diskreter digitaler Abtastungen ($n\tau + k\tau$) mit einer Abtastdauer $\tau$ dargestellt werden, dadurch gekennzeichnet, daß die Schaltung eine Anzalh von Teilschrittverzögerungs-(FSD)-Digitalinterpolationsfilter (86, 87, 88), die je zum Interpolieren des Wertes des zweiten Eingangssignals zu einem anderen vorgegebenen Zeitpunkt zwischen den diskreten Abtastungen dienen, und eine Anzahl diskreter Zeitkorrelationsschätzfunktionsschaltungen (89...93) in Kaskadenschaltung mit den Interpolationsfiltern enthält, so daß jede der Schaltungen (89...93) die Korrelation zwischen dem ersten Signal und einem Ausgangssignal eines der Interpolationsfilter bestimmt.

2. Korrelatorschaltung nach Anspruch 1, dadurch gekennzeichnet, daß das erste Eingangssignal einem ersten Eingang jeder der Korrelationsschätzer zugeführt wird, das zweite Eingangssignal an den Eingang jedes der FSD-Interpolationsfilter gelegt wird, und das Ausgangssignal jedes der FSD-Filter an den zweiten Eingang eines entsprechenden diskreten Zeitkorrelationsschätzers gelangt.

3. Korrelatorschaltung nach Anspruch 1, dadurch gekennzeichnet, daß die Schaltung weiter noch erste fest eingestellte Verzögerungsmittel mit einer Laufzeit Kт, wobei das erste Eingangssignal an den Eingang des ersten Verzögerungsmittels gelangt, und zweite fest eingestellte Verzögerungsmittel mit einer Laufzeit Kт enthält, wobei das zweite Eingangssignal an den Eingang des zweiten Verzögerungsmittels gelangt, jedes der FSD-Interpolationsfilter zum Empfangen von Abtastungen des zweiten Eingangssignal und zum Erzeugen einer Schätzung des Wertes des zweiten Signals zu einem anderen Zeitpunkt zwischen den diskreten Abtastzeitpunkten des Signals angeschlossen ist, so daß jedes der FSD-Filter den Wert des zweiten Signals zu einem separaten Zeitpunkt zwischen den diskreten Abtastzeitpunkten abschätzt, und jede der diskreten Zeitkorrelationsschätzfunktionsschaltungen mit einem ersten Eingang zum Empfangen verzögerter Abtastungen des ersten Signals vom Ausgang des ersten fest eingestellten Verzögerungsmittels und mit einem zweiten Eingang zum Empfangen eines der Abschätzungen des Wertes des zweiten Signals von einem entsprechenden Ausgang entweder des zweiten Verzögerungsmittels oder eines der Interpolationsfilter angeschlossen ist, um daraus einen Schätzungswert der Kreuzkorrelation zwischen den den ersten und zweiten Eingängen zugeführten Eingangssignalen zu erzeugen.

4. Korrelatorschaltung nach einem der Ansprüche 1, 2 oder 3, dadurch gekennzeichnet, daß die diskreten Zeitkorrelationsschätzfunktionsschaltungen Einbit-Korrelatoren sind.

5. Korreltorschaltung nach einem der vorangehenden Ansprüche, dadurch gekennzeichnet, daß die Teilschrittverzögerungs-Interpolationsfilter zum Interpolieren des Werts des zweiten Eingangssignals in einer Gruppe von Intervalle ausgelegt sind, die Teile der Abtastdauer $\tau$ nach den diskreten Abtastzeit-punkten des zweiten Signals sind.

6. Korrelatorschaltung nach Anspruch 5, dadurch gekennzeichnet, daß darin N-1 Teilschrittverzögerungs-Interpolationsfilter vorgesehen sind, die zum Interpolieren des Wertes des zweiten Eingangssignals bei Intervallen von

$$\frac{K\tau}{N}$$

nach den diskreten Abtastzeitpunkten des zweiten Signals dienen, worin N eine ganze Zahl größer als 1 und k = 1, 2, 3...N-1 sind.

7. Anordnung zum Bestimmen von Bewegungsparametern in einem Medium durch Ultraschall-Impulsechomessungen, mit wenigstens einem Ultraschallwandler, Mitteln zum Anregen der Aussendung eines pulsierten Echoschallsignals vom Wandler bei einer Impulswiederholungsfrequenz F = 1/T, Mitteln zum Empfangen von Ultraschallechos, die vom Medium zurück zum Wandler geworfen werden, und zum Verarbeiten derartiger Signale in einem Digitalverarbeitungskanal, der einwirkt auf diskrete Abtastungen von A-Zeilen-Signalen aus dem Wandler, und die in Kaskadenschaltung Mittel zum Unterdrücken von Signalen in den A-Zeilen enthalten, die aus festen Bereichen in dem Medium herrühren, Flußparameter-schätzungsmittel, Diskriminatormitteln sowie Mitteln zum Anzeigen der Schätzungswerte von Flußparametern, dadurch gekennzeichnet, daß die Flußparameterschätzungsmittel eine Korrelatorschaltung nach einem oder mehreren der vorangehenden Ansprüche enthalten.

FIG. 1

FIG. 3

FIG. 2

FIG. 4

$g(t)$

1

$-\tau$   0   $\tau$   $t \longrightarrow$

# FIG.5

$\tau$   $t \longrightarrow$

# FIG.6

$g(t) = \dfrac{SIN(\pi t/\tau)}{\pi t/\tau} = SINC(t/\tau)$

1

$\tau$   $2\tau$   $3\tau$   $t \longrightarrow$

# FIG.7

$\tau$   $t \longrightarrow$

# FIG.8

FIG.9

FIG.10a

FIG.10b

FIG.11

FIG.12

FIG.13

FIG.14a

FIG.14b